Europäisches Patentamt

European Patent Office

Office européen des brevets

(11) Veröffentlichungsnummer: **0 355 930**
**A2**

## (12) EUROPÄISCHE PATENTANMELDUNG

(21) Anmeldenummer: 89202135.3

(22) Anmeldetag: 04.08.89

(51) Int. Cl.⁴: **A61F 5/04**

(30) Priorität: 05.08.88 DE 3826704
30.12.88 DE 3844381

(43) Veröffentlichungstag der Anmeldung:
28.02.90 Patentblatt 90/09

(84) Benannte Vertragsstaaten:
**ES GR**

(71) Anmelder: **Habermeyer, Peter, Dr.**
**Oberföhringer Strasse 27**
**D-8000 München 81(DE)**

(72) Erfinder: **Habermeyer, Peter, Dr.**
**Oberföhringer Strasse 27**
**D-8000 München 81(DE)**

(74) Vertreter: **Dipl.-Phys.Dr. Manitz**
**Dipl.-Ing.Dipl.-Wirtsch.-Ing. Finsterwald**
**Dipl.-Phys. Rotermund Dipl.-Chem.Dr. Heyn**
**B.Sc.(Phys.) Morgan**
**Robert-Koch-Strasse 1**
**D-8000 München 22(DE)**

(54) **Vorrichtung zur umschliessenden Fixierung von Extremitäten.**

(57) Es wird eine Vorrichtung zur umschließenden Fixierung von Extremitäten beschrieben, bei der ein doppelwandiges, zu einer Hülse verformbares, vakuumdicht ausgebildetes und mit einem Evakuierungsventil versehenes Manschettenkissen verwendet wird, in dessen zwischen den beiden Wänden gelegenem Innenraum eine Vielzahl von losen Füllkörperteilchen vorgesehen ist und das durch Evakuierung zu einem in sich steifen, stabilen Gebilde verfestigbar ist.

Fig. 1

EP 0 355 930 A2

## Vorrichtung zur abschliessenden Fixierung von Extremitäten

Die Erfindung betrifft eine Vorrichtung zur umschließenden Fixierung von Extremitäten und Extremitätenbereichen, insbesondere zur Behandlung von Extremitätenfrakturen 1m Bereich von Unterschenkel, Oberschenkel, Unterarm und Oberarm sowie zur Verwendung in Skischuhen und dergleichen.

Zur Behandlung von Extremitätenfrakturen werden üblicherweise Gipsverbände oder deren moderne Varianten in Form von Kunststoffverbänden angelegt. Das Anlegen derartiger Verbände erfordert nicht nur ein erhebliches Geschick und einen entsprechenden Aufwand für das Personal, sondern führt häufig auch dazu, daß durch Kanten oder Vorsprünge an der Innenseite des Gips- oder Plastikverbandes für den Patienten äußerst störende Druckstellen entstehen. Nach der Reposition der jeweiligen Fraktur ist der Patient der schmerzhaften Phase des Nachwickelns der Binden und des Wartens auf das Abbinden des Gipses ausgesetzt. Schließlich ist es bei den bekannten Gips- oder Plastikverbänden im Regelfall erforderlich, einen Wechsel dieses Gips- oder Plastikverbandes vorzunehmen, wenn es im Laufe der Frakturbehandlung zu einer Abschwellung der betreffenden Gliedmaße kommt. Auch dies ist ein aufwendiger und für den Patienten wiederum unangenehmer Vorgang.

Aufgabe der Erfindung ist es, eine Vorrichtung der eingangs angegebenen Art in der Weise auszubilden, daß die geschilderten Nachteile von Gips- und Plastikverbänden vermieden werden können und eine einfach und schnell vornehmbare, zu keinerlei Druckstellen führende umschließende Fixierung von Extremitäten möglich ist, die praktisch unverzüglich wirksam ist, einfach gelöst und sofort wieder neu angelegt werden kann. Außerdem soll die Vorrichtung eine exakte und dennoch druckfreie Abstützung innerhalb Skischuhen und dergleichen gewährleisten.

Diese Aufgabe wird nach der Erfindung im wesentlichen gelöst durch ein doppelwandiges, zu einer Hülse verformbares, vakuumdicht ausgebildetes und mit zumindest einem Evakuierungsventil versehenes Manschettenkissen, in dessen zwischen den beiden Wänden gelegenem Innenraum eine Vielzahl von relativ zueinander beweglichen Füllkörpern vorgesehen ist.

Das zu einer Hülse verformbare und damit eng und passend an den zu behandelnden Gliedabschnitt anlegbare Manschettenkissen wird bei einer innerhalb kurzer Zeit durchführbaren Evakuierung in der während des Anlegevorgangs geschaffenen Form hart und formstabil, so daß eine sehr feste, an die jeweilige Extremität gut anmodellierte Hülse entsteht, die im Bereich der Haut keinerlei Druckstellen erzeugen kann, da bei der Verfestigung dieses Gebildes kein radial nach innen gerichteter Druck entsteht und an der Innenseite auch keine Kanten oder Vorsprünge gebildet werden können.

Vorzugsweise ist das Manschettenkissen an seinen Längsrandbereichen mit Hülsen-Verschlußorganen versehen, die insbesondere aus streifenförmigen Klettverschlußteilen bestehen. Damit läßt sich sehr einfach und schnell aus dem zunächst flachen, schmiegsamen und leicht verformbaren Manschettenkissen eine Hülse absolut korrekter Paßform erzeugen und zunächst über den Klettverschluß festlegen. Nach erfolgter Evakuierung liegt dann eine Hülse vor, die in sich verfestigt in ihrer Form stabilisiert ist.

Das Manschettenkissen besteht aus einem für Röntgenstrahlen durchlässigen Material, insbesondere aus einem im wesentlichen nicht dehnbaren Kunststofffolienmaterial in haut freundlicher Ausführung oder entsprechender hautseitiger Ausrüstung.

Durch die Wahl eines im wesentlichen nicht dehnbaren Folienmaterials wird wesentlich zur Verfestigung der evakuierten Hülse beigetragen, da die eng an den Füllkörperteilchen anliegende Folie nicht gedehnt werden kann und damit in Verbindung mit den zwischen den Füllkörpern geschaffenen Reibschlußkontakten ein praktisch starres Gebilde entsteht.

Die Füllkörperteilchen des flachen, zu einer Hülse verformbaren Manschettenkissens bestehen zumindest zum Teil aus einander flächig überlappenden und im aneinandergepreßten Zustand kraftschlüssig verbundenen Teilchen, die vorzugsweise zumindest im wesentlichen inkompressibel sind. Hinsichtlich der verwendbaren Füllkörperteilchen besteht eine große Auswahl, aber es ist bei dieser Auswahl auch darauf zu achten, daß möglichst leichte Teilchen Verwendung finden, die aber in ihrem Zusammenwirken, d.h. im aneinandergepreßten und zwischen den Wänden des Manschettenkissens eingeschlossenen Zustand zu einer hohen Gesamtfestigkeit führen.

In dem Manschettenkissen kann zumindest ein den Durchtritt von Extensionsdrähten, Schläuchen und dergleichen ermöglichender Perforationsbereich mit vakuumdichten Rändern vorgesehen sein, so daß ohne Schwierigkeiten auch eine Drahtextensionsbehandlung im Bereich des Unterschenkels erfolgen kann.

Gemäß einer bevorzugten Ausführungsform der Erfindung ist der schmiegsam ausgebildeten und durch Unterdruck verfestigbaren Hülse eine aus stabilem Kunststoff bestehende Schutzhülse zugeordnet, welche die dann eine Innenhülse darstellende Vakuumhülse umschließt. Diese Schutzhülse kann zusätzlich eine erwünschte Versteifung des Verbands erbringen.

Diese Kunststoffhülse ist der Form der jeweiligen Extremität zumindest im wesentlichen angepaßt und von schalenartigem Aufbau oder so flexibel ausgeführt, daß ein Anlegen über einen ventralen Schlitz erfolgen kann. Durch diese zusätzliche Stabilisierung aufgrund der äußeren Kunststoffhülse ist es ohne weiteres möglich, den gesamten Hülsenapparat auch nach Art eines Gehgipses auszubilden.

Vorzugsweise besitzt die Kunststoffhülse eine innere Beschichtung in Form einer hartelastischen Polsterung, welche die unterschiedlich großen Raumbereiche zwischen Innenhülse und Außenhülse füllt und gleichzeitig bei Aufprallbelastungen Stoßpufferfunktion übernimmt.

Diese äußere Kunststoffhülse kann auch über Klettverschlüsse fixiert werden, so daß stufenfreie Arretierungen möglich sind.

Die erfindungsgemäße Vorrichtung kann auch nach Art eines langen Oberschenkelgipses oder als stabile, sich vom Sprunggelenk bis zum oberschenkelansatz erstreckende Hülse ausgebildet werden, und es bereitet auch keine Schwierigkeit, eine Ausgestaltung nach Art eines Unterschenkelliege - oder -gehgipses vorzunehmen. Weitere Anwendungen sind beispielsweise möglich bei Radiusfrakturen zur Ruhigstellung von Hand und Unterarm sowie als Oberarm-Brace zur Ruhigstellung von Oberarmfrakturen, und es ist natürlich auch eine Verwendung nach Art eines Oberarmgipses zur Ruhigstellung von Oberarm, Unterarm und Handgelenk möglich.

Besonders vorteilhaft ist es, die Vorrichtung nach der Erfindung in Verbindung mit Schuhen, insbesondere Skischuhen zu verwenden, da im Gegensatz zu Ausschäumvorgängen oder aufblasbaren Einlagen eine sehr individuelle, auf die jeweiligen Momentangegebenheiten abstellbare Anpassung der Einlage möglich ist, und zwar unter absoluter Vermeidung von auf den Fuß Druck ausübenden Bereichen.

Besonders vorteilhafte Ausgestaltungen dieser Verwendungsmöglichkeit der Erfindung sind in den Ansprüchen 11 bis 17 angegeben.

Die Erfindung wird anhand eines Ausführungsbeispiels und unter Bezugnahme auf die Zeichnung näher erläutert; in der Zeichnung zeigt:

Fig. 1 eine schematische Längsschnittdarstellung eines mit einer Vorrichtung nach der Erfindung versehenen Unterschenkels,

Fig. 2 eine schematische Teil-Querschnittsdarstellung einer Ausführungsform der Vorrichtung nach der Erfindung, und

Fig. 3 eine Schnittdarstellung entsprechend der Linie A-A von Fig. 1.

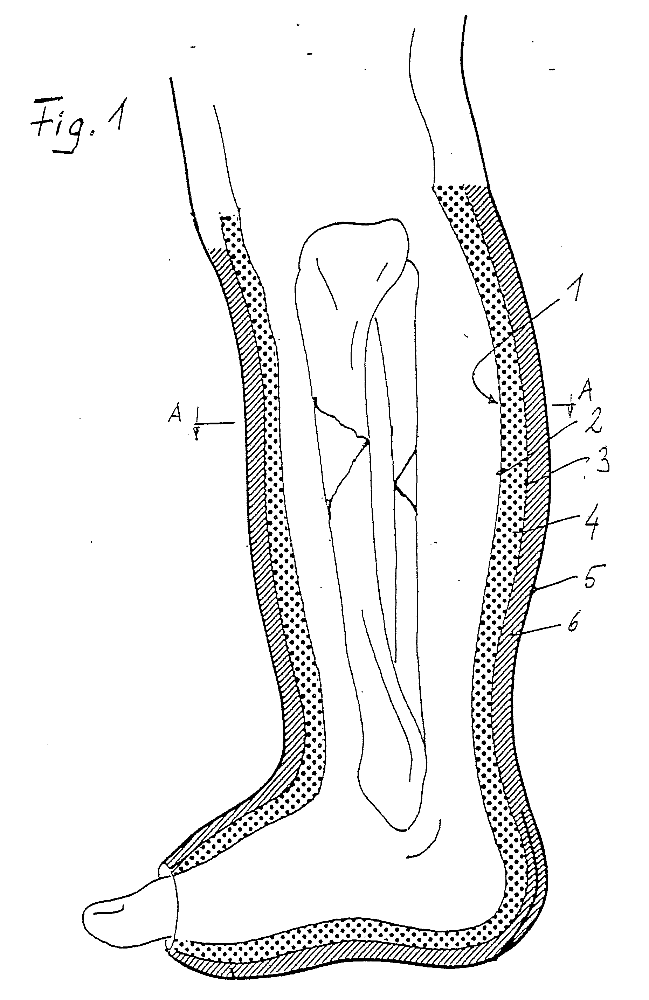

Fig. 1 zeigt einen Unterschenkel, an dem anstelle eines herkömmlichen Gipsverbandes eine Vorrichtung nach der Erfindung angebracht ist.

Diese Vorrichtung umfaßt ein zu einer Hülse geformtes Manschettenkissen 1, das aus einer Innenwand 2 und einer Außenwand 3 besteht, die vakuumdicht ausgebildet und an ihren Randbereichen vakuumdicht miteinander verbunden, insbesondere verschweißt sind, so daß ein Innenraum entsteht, der mit einer Vielzahl einzelner Füllkörperteilchen angefüllt ist. Auf diese Weise entsteht ein sehr flaches, kissenartiges und sehr schmiegsames Gebilde, das um den jeweiligen Gliedmaßen abschnitt herumgelegt und insbesondere mittels eines Klettverschlusses bündig und formschlüssig arretiert werden kann.

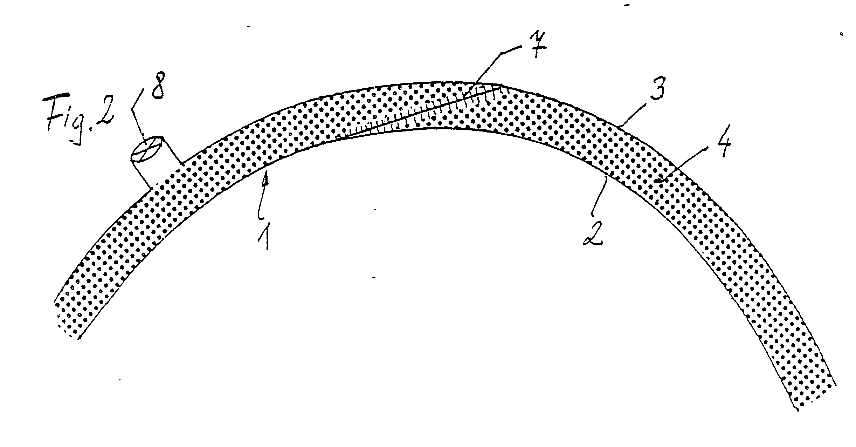

Die Teilschnittdarstellung nach Fig. 2 zeigt das Manschettenkissen 1 im Bereich seiner Verbindung zu einem Hülsengebilde, wobei die Randbereiche des Manschettenkissens mit Klettverschlußelementen 7 versehen sind, so daß durch entsprechende Überlappung das Hülsengebilde auch schon dann in seiner Grundform festgelegt werden kann, wenn der Absaugvorgang noch nicht stattgefunden hat. Um eine Evakuierung des Raumes zwischen den beiden Wandungen 2 und 3 zu ermöglichen, ist zumindest ein Evakuierungsventil 8 vorgesehen.

Nach der Arretierung des plastisch gut anmodellierten Hülsengebildes und nach genauer Einrichtung des frakturierten Unterschenkels und der Röntgenkontrolle wird durch Anschalten einer Absaugpumpe innerhalb sehr kurzer Zeit der erforderliche hohe Unterdruck im Manschettenkissen erzeugt, der dazu führt, daß die Füllkörperteilchen untereinander aufgrund des Differenzdrucks zur Atmosphäre durch die Wandungen 2 und 3 fest aneinander gepreßt werden, so daß aufgrund des Kraft-und Formschlusses zwischen den Wandungen 2 und 3 und den Füllkörperteilchen ein steifes Gebilde entsteht, das den gebrochenen Unterschenkel schient und stabilisiert.

Zum Schutz dieses unter Vakuum stehenden Gebildes und zur weiteren Stabilisierung wird eine äußere Kunststoffhülse 5 angelegt, die in etwa die Form des Unterschenkels aufweist und schalenartig ausgebildet oder so flexibel ist, daß über einen ventralen Schlitz ein Anlegen möglich ist. An der Innenseite der äußeren Kunststoffhülse 5 ist eine hartelastische Polsterung 6 vorgesehen, welche die Zwischenräume zwischen der verfestigten Innenhülse 1 und der Außenhülse 5 ausfüllt und zugleich als Stoßpuffer bei Aufprallbelastungen wirkt und z.B. beim Gehen Widerstand leistet.

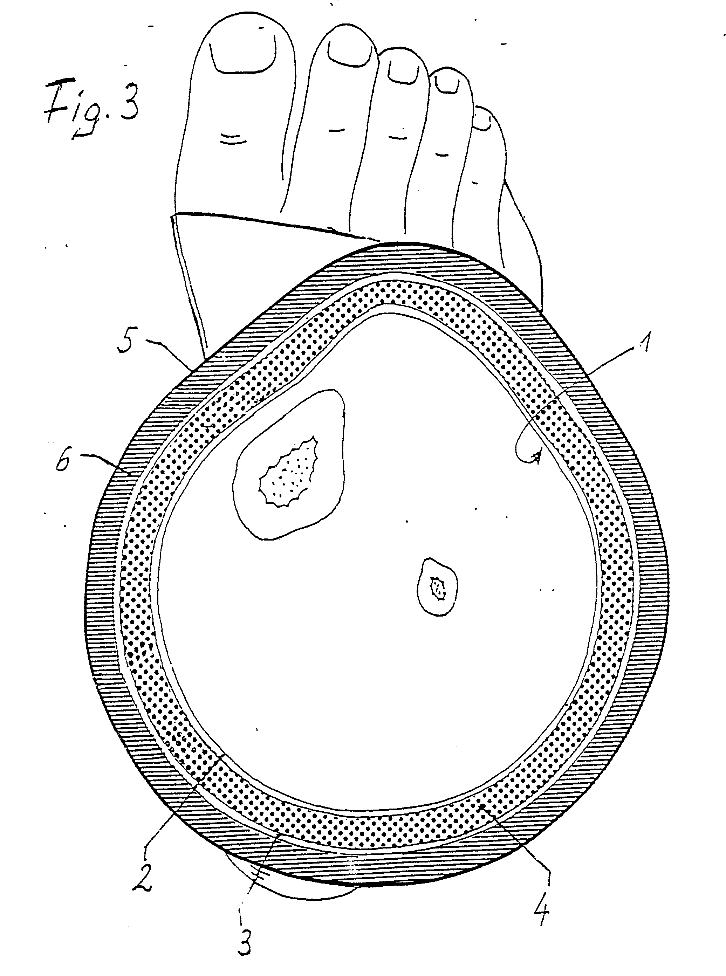

Die Schnittansicht nach Fig. 3 läßt erkennen, daß von der verfestigten Innenhülse 1 auf den Unterschenkel kein störender, insbesondere kleinflächiger Druck ausgeübt werden kann, da die Verfestigung dieser Hülse beim Anlegen einer Unterdruckquelle zu einer Vergrößerung des lichten Innendurchmessers führt, was zur Folge hat, daß zwischen dem Unterschenkel und der verfestigten Innenhülse ein geringer Spalt entsteht.

Neben den bereits erwähnten Vorteilen ist darauf hinzuweisen, daß die Vorrichtung nach der Erfindung sterilisierbar ausgeführt werden kann, damit häufig wiederverwendbar ist und zu einer Senkung der Behandlungskosten beiträgt. Es ist insbesondere auch eine Verwendung bei der Behandlung offener Frakturen möglich, weil ein Verbandswechsel problemlos durch Aufhebung des Unterdrucks möglich ist und damit die Wunde gut zugänglich wird. Ein erneutes Anlegen ist innerhalb kurzer Zeit problemlos möglich. Dies führt auch dazu, daß sogar eine Abnahme des Verbandes aus hygienischen Gründen, z.B. zum Duschen, vertretbar ist und vor allem auch nach Lockerung der Klettverschlüsse und dann erfolgende erneute Evakuierung eine engere Anlegung des Verbandes möglich ist, wenn es im Verlaufe der Frakturbehandlung zu einer Abschwellung des zu behandelnden Gliedes kommt. Der sonst erforderliche Gipswechsel entfällt auf diese Weise.

Die Erfindung ist vorteilhaft im Bereich der konservativen Behandlung von Brücken sowie Kontusionen und Distorsionen der oberen und unteren Extremität im Fachgebiet Chirurgie, Unfallchirurgie und Orthopädie verwendbar.

Ein praktisch besonders interessanter Anwendungsbereich der Erfindung ergibt sich im Zusammenhang mit Schuhen, insbesondere Skischuhen, wo es möglich ist, eine differenzierte Anpassung des Schuhs an den jeweiligen Fuß zu erreichen, und zwar ohne Schaffung von Druckbereichen, wie dies bei bisher üblichen Einlagen häufig der Fall ist.

Bei Skischuhen ist es möglich, den Innenschuh insgesamt als durch Unterdruck verfestigbares Teil auszubilden, aber dies stellt keine Notwendigkeit dar. In vielen Fällen ist es von Vorteil, die bereits erwähnten kritischen Bereiche mit den erfindungsgemäßen Unterdruckkissen auszustatten, wobei diese Kissen in den Innenschuh integriert werden können.

Wenn der Innenschuh sehr dünn gehalten werden soll, ist es auch möglich, die Unterdruckkissen unmittelbar am Außenschuh bzw. der festen Schale anzubringen, wobei die Schalenwand gleichzeitig eine Kissenwand bilden könnte. Erreichen die Unterdruckkissen eine bestimmte Größe, bei der es möglich ist, daß sich die Füllkörper in ungewünschter Weise verlagern, so können Maßnahmen getroffen werden, um die Füllkörper zumindest in etwa in ihrer Grundposition zu halten, ohne jedoch dabei die leichte Verformbarkeit des Kissens zu beeinträchtigen, solange dieses noch nicht evakuiert ist.

Von Vorteil ist es auch, als Füllkörper Teilchen bzw. Stoffe zu verwenden, die eine hohe Isolationseigenschaft haben, da sich damit gerade die besonders gefährdeten Bereiche innerhalb eines Skischuhs besonders vorteilhaft vor Kälte schützen lassen.

## Ansprüche

1. Vorrichtung zur umschließenden Fixierung von Extremitäten und Extremitätenbereichen, insbesondere zur Behandlung von Extremitätenfrakturen im Bereich von Unterschenkel, Oberschenkel, Unterarm und Oberarm, sowie zur Verwendung in Skischuhen und dergleichen,
**gekenneichnet** durch
ein doppelwandiges, zu einer Hülse verformbares, vakuumdicht ausgebildetes und mit zumindest einem Evakuierungs ventil (8) versehenes Manschettenkissen (1), in dessen zwischen den beiden Wänden (2, 3) gelegenem Innenraum eine Vielzahl von relativ zueinander beweglichen Füllkörpern (4) vorgesehen ist.

2. Vorrichtung nach Anspruch 1,
dadurch **gekenneichnet,**
daß das vorzugsweise aus einem für Röntgenstrahlen durchlässigen, im wesentlichen nicht dehnbaren Folienmaterial bestehende Manschettenkissen (1) an seinen Längsrandbereichen mit Hülsen-Verschlußorganen (7) versehen ist, die insbesondere aus Streifenförmigen Klettverschlußteilen, Schnürverschlüßen und dergleichen bestehen.

3. Vorrichtung nach Ansrpuch 1,
dadurch **gekennzeichnet,**
daß die Füllkörper (4) aus losen Füllkörperteilchen und insbesondere zumindest zum Teil aus einander flächig überlappenden und im aneinandergepreßten Zustand kraftschlüssig miteinander verbundenen Teilchen bestehen.

4. Vorrichtung nach einem der vorhergehenden Ansprüche,
dadurch **gekennzeichnet,**
daß die Füllkörper (4) zwischen Pufferschichten, insbesondere in Form von Vlieslagen, angeordnet und insbesondere zumindest zum Teil im wesentlichen inkompressibel sind.

5. Vorrichtung nach einem der vorhergehenden Ansprüche,
dadurch **gekenneichnet,**
daß in dem innenseitig vorzugsweise mit einer hartelastischen Polsterung (6) versehenen Manschettenkissen (1) zumindest ein den Durchtritt von Extensionsdrähten, Schläuchen und dergleichen ermöglichender Perforationsbereich mit vakuumdichten Rändern vorgesehen ist.

6. Vorrichtung nach einem der vorhergehenden Ansprüche,
dadurch **gekenneichnet,**

daß der schmiegsam ausgebildeten und durch Unterdruck verfestigbaren Hülse (1) eine aus stabilem Kunststoff bestehende Schutzhülse (5) zugeordnet ist, die die durch Unterdruck verfestigbare Hülse (1) umschließt, wobei die Schutzhülse (5) vorzugsweise mit der Hülse (1) bereichweise verbunden und insbesondere in ihrem Durchmesser über Klettverschlüsse stufenlos verstellbar ist.

7. Vorrichtung nach einem der vorhergehenden Ansprüche,
dadurch **gekennzeichnet,**
daß die Kunststoffhülse (5) der Form der jeweiligen Extremität zumindest im wesentlichen angepaßt ist und einen schalenartigen, insbesondere aufklappbaren Aufbau aufweist oder so flexibel ausgeführt ist, daß ein Anlegen über einen Schlitz erfolgen kann.

8. Vorrichtung nach einem der vorhergehenden Ansprüche,
dadurch **gekennzeichnet,**
daß das Manschettenkissen (1) aus einem Kunststoffmaterial mit integriertem oder daran befestigtem, zumindest im wesentlichen nicht dehnbaren Festigkeitsträger, insbesondere in Netzform, besteht und vorzugsweise an seiner innenliegenden Seite mit einem einen Luft- und Feuchtigkeitsaustausch gewährleistenden Material beschichtet ist.

9. Vorrichtung nach einem der vorhergehenden Ansprüche,
dadurch **gekenneichnet,**
daß zumindest zwei Arten von Füllkörperteilchen (4) vorgesehen sind, wobei eine Art überwiegend zur Festigkeitserzielung beiträgt und die andere, insbesondere feinkörnige Art Pufferfunktion besitzt.

10. Vorrichtung nach einem der vorhergehenden Ansprüche,
dadurch **gekenneichnet,**
daß das Manschettenkissen in mehrere getrennt evakuierbare Bereiche unterteilt ist.

11. Vorrichtung nach einem oder mehreren der vorhergehenden Ansprüche in Verbindung mit einem Schuh, insbesondere Skischuh,
dadurch **gekennzeichnet,**
daß zwischen der den Fuß umschließenden Hülle und dem Fuß zumindest auf Teilbereichen wenigstens ein verformbares, vakuumdicht ausgebildetes und mit zumindest einem verschließbaren Evakuierungsanschluß versehenes Kissen vorgesehen ist, in dessen Innenraum ein bei Unterdruck verfestigbarer Füllstoff vorgesehen ist.

12. Vorrichtung nach einem der vorhergehenden Ansprüche,
dadurch **gekennzeichnet,**
daß die Füllkörper innerhalb des jeweiligen Kissens in ihrer Position im wesentlichen festgelegt, insbesondere zur Lagepositionierung lose miteinander verbunden sind und/oder das jeweilige Kissen zur positionierung der Füllkörper in Einzelbereiche unterteilt ist, wobei vorzugsweise die Einzelbereiche gemeinsam evakuierbar sind.

13. Vorrichtung nach einem der vorhergehenden Ansprüche,
dadurch **gekenneichnet,**
daß der Sohlenbereich des Schuhs frei von durch Unterdruck verfestigbaren Kissen ist und daß die verformbaren und durch Unterdruck verfestigbaren Kissen im Fersenbereich, und/oder im Sprunggelenkbereich und/oder im Achillessehnenbereich und/oder im Außen- oder Innenballenbe reich bevorzugt angeordnet sind.

14. Vorrichtung nach einem der vorhergehenden Ansprüche,
dadurch **gekennzeichnet,**
daß die Füllung und/oder Ausdehnung und/oder Dicke der jeweiligen Kissen innerhalb eines Schuhs zumindest zum Teil unterschiedlich gewählt ist.

15. Vorrichtung nach einem der vorhergehenden Ansprüche,
dadurch **gekennzeichnet,**
daß die Kissen in den Schuhaufbau integriert sind und/oder Wandlungsteile des Schuhs gleichzeitig eine Kissenwandung bilden.

16. Vorrichtung nach einem der vorhergehenden Ansprüche,
dadurch **gekenneichnet,**
daß die Kissen in den Innenschuh eines Skischuhs zumindest zum Teil integriert sind und daß insbesondere zumindest ein Teil der Kissen mit der Außenschale des Schuhs fest verbunden bzw. in die Außenschale integriert ist.

17. Vorrichtung nach einem der vorhergehenden Ansprüche,
dadurch **gekenneichnet,**
daß im oberen Randbereich des Innenschuhs oder der Schale ein zu einer Hülse verformbares, insbesondere mittels eines Verschlusses in Hülsenform festlegbares Unterdruckkissen vorgesehen ist, das eine funktionelle Schaftverlängerung bildet und mit dem Schuh entsprechend verbunden ist.

Fig. 1

Fig.2

*Fig.3*